# EUROPEAN PATENT SPECIFICATION

(11) **EP 0 942 699 B1**
(45) Date of publication and mention of the grant of the patent: **18.09.2002**
(21) Application number: 97913604.1
(22) Date of filing: 11.11.1997
(51) Int. Cl.: A61F 13/15

(54) **ABSORBENT ARTICLE WITH A REINFORCED HUMP**
ABSORBIERENDER ARTIKEL MIT VERSTÄRKTEM ERHÖHTEM MITTELTEIL
ARTICLE ABSORBANT A BOSSE RENFORCEE

(30) Priority: 15.11.1996 SE 9604225
(43) Date of publication of application: 22.09.1999
(73) Proprietor: SCA Hygiene Products AB, 405 03 Göteborg (SE)
(72) Inventor: GUIDOTTI, Ted, S-412 67 Göteborg (SE); JOHANSSON, Anette, S-416 85 Göteborg (SE); BJÖRKLUND, Camilla, S-412 62 Göteborg (SE); WIDLUND, Urban, S-435 34 Mölnlycke (SE)
(74) Representative: Romare, Laila Anette
(86) International application number: SE9701884
(87) International publication number: WO98022060

(56) References cited:
- WO-A-95/31165
- WO-A-96/26699
- US-A- 5 545 156
- US-A- 5 558 656

## Description

### TECHNICAL FIELD:

The invention relates to an absorbent article intended for female users, such as a sanitary napkin, a panty-liner or an incontinence guard, with such a shape and size that it may be substantially accommodated in the crotch region of a pair of panties and with a longitudinal direction, a transverse direction and a thickness direction, whereby the article exhibits a liquid-pervious surface, and a liquid-impervious surface, and further an absorbent body arranged between the two surfaces, and wherein a hump is arranged at the liquid-pervious surface of the article.

### BACKGROUND OF THE INVENTION:

Absorbent articles, of the kind which are supported by the underclothing of a user, have a comparatively small size. Thereby, a common problem is leakage of body fluid past the longitudinal side edges of the article. Neither is it unusual for leakage past the end edges of the article to occur. Thereby, a particular problem is leakage backwards which happens more often than is acceptable when the user is lying down. It is, of course, extremely important to reduce all forms of leakage of body fluid, since this results in a number of nuisances such as embarrassing stains, additional laundry, and sometimes also that the soiled clothing or bedding has to be discarded.

A contributing cause of edge leakage is that the absorbent article is deformed during use, when the article is squeezed between the thighs of the user. Thereby, wrinkling arises, in an uncontrolled manner, in both the absorbent core of the article and in its cover material. Such wrinkling results in the formation of channels on the surface of the article, in which body fluid may seep out past the side edges. Furthermore, the compression of the article implies that the area accessible for liquid acquisition is reduced, whereby the risk of body fluid ending up outside the article is significant.

In connection with the previously known absorbent articles, a further problem is that the side edges of the articles risk being folded in across the liquid-pervious surface of the article because of the action from the body forces which arise when the user moves about. The area accessible for liquid acquisition is drastically reduced also by such folding in, since only a narrow liquid-pervious region is thereby left between the inwardly-folded side edges.

One way of reducing the risk of edge leakage, caused by deformation of the article during use, is to provide the article with a pre-formed hump, which during use is intended to be in contact with the genitals of the user. Excreted body fluid may in this way be caught as soon as it leaves the body of the user, and immediately be absorbed into the article without flowing out over its surface.

Previously known absorbent articles provided with a liquid-receiving hump are, however, marred by a number of disadvantages.

A common way of creating a hump has been to simply build it up by arranging a larger quantity of absorbent material within the area of the hump. Since the most commonly occurring absorbent material is so called cellulose fluff pulp, such a hump, however, collapses and loses its shape when it is wetted. In order to achieve a hump which is sufficiently large also in a wet state, a hump consisting of cellulose fluff pulp must comprise so much absorbent material that it is far too high, hard and uncomfortable to wear in a dry state.

In EP 0 335 252 and EP 0 335 253 it has been suggested to provide an absorbent article with a deformation element. The deformation element is affected by the transverse compressive forces between the thighs of a user. The purpose of the deformation element is that this during use should bulge a portion of the article in a direction towards the body of the user. However, it is impossible to completely control, or anticipate, the shape the article will adopt for each individual user. Furthermore, it is not possible to ensure the contact between the body of the user and the surface of the article, since the degree of bulging is completely determined by how much the article is compressed in the transverse direction.

A similar sanitary napkin including a shaping component is disclosed in US-A-5 558 656.

Thus, there remains a need for a leakage-proof absorbent article which has a predictable shape, both before and during use, and which maintains its shape independently of the movements of the user and of the wetting the article is subjected to.

An article designed according to the invention, of the kind mentioned in the introduction, is primarily characterized in that the hump comprises a stiffening element which, both in a wet and in a dry state, counteracts compression in the thickness direction of the article and which extends between the liquid-pervious surface and the liquid-impervious surface.

Since the hump of the article comprises a stiffening element, which counteracts deformation of the hump in the height direction, a good contact between the hump and the body of the user is ensured during use. In this manner, it is also possible to form a comparatively narrow and high hump which maintains its shape also when it is wet or subjected to pressure forces, for example when the user is sitting. Furthermore, the stiffening element may be utilized as a folding means for absorbent layer material being part of the hump and for the liquid-pervious surface material of the article, so that the hump is imparted a narrow, well-defined ridge-shape.

The height of the hump above the surface of the absorbent article must be adapted so that during use good contact between the body of the user and the hump is ensured. However, since the hump by means of the stiffening means is so hard or stiff that it is essentially unyielding to the compressive forces it is subjected to during use, it is important that the hump is not so high that it presses against the body of the user and thereby causes discomfort during use. Furthermore, it is necessary to ensure that the hump does not chafe against the sensitive soft parts in the crotch region of the user. Thereby, it has been found that a hump which at its highest portion projects at least 5 mm above the surface of the article, but no more than 20 mm, fulfils the requirements of both good body contact and high user comfort.

Furthermore, a hump in accordance with the invention should be relatively narrow, suitably between 2 mm and 25 mm at the base, and preferably between 12 mm and 16 mm. At the top, the hump is suitably between 3 mm and 10 mm wide, and preferably between 4 mm and 6 mm. The hump is designed with a substantially triangular cross-section and, consequently, is always wider at the base than at the top. Since the hump is comparatively narrow, it can without any discomfort to the user protrude slightly in between the labia pudendi of the user. Thereby, it is an advantage if the hump slightly separates the labia pudendi, since the liquid transfer from the user to the article is thus facilitated.

By utilizing a very narrow stiffening element, for example a stiffening element consisting of a thin material plate, the stiffening element constitutes no noticeable obstacle for liquid reception and absorption into the hump of the article. Thereby, suitable stiffening elements are, for instance, manufactured from a stiff plastic layer, with a thickness between 0.3 mm and 2 mm. When thinner plastic layers are used, with a thickness between 0.2 mm and 0.6 mm, the stiffening element may be present in the form of a doubled piece of the plastic layer. Such an embodiment increases the stability of the stiffening element with respect to lateral movements, without influencing the flow of liquid into the absorbent article to any larger extent.

At the back portion of the hump, it should have such a shape that it closely conforms to the body shape of the user in the region behind the vaginal opening. Thereby, it is avoided that body fluid flows backwards in the furrow between the buttocks of the user and leaks out from the article. Suck leakage backwards is particularly troublesome when the user is lying down. In order to obtain an optimum combination of leakage-security and user comfort, the hump is suitably somewhat lower and narrower at its end portions.

### BRIEF DESCRIPTION OF THE DRAWINGS:

In the following, the invention will be described in greater detail with reference to the figures which are shown in the attached drawings.

Thereby:
- Fig. 1: shows a plan view of a sanitary napkin according to a first embodiment, seen from the side which in use is facing the user,
- Fig. 2: shows a section along the line II-II through the sanitary napkin in Fig. 1,
- Fig. 3-6: show different designs of the stiffening element according to the invention,
- Fig. 7: shows a sanitary napkin according to a second embodiment, seen from the side which in use is facing the user, and
- Fig. 8: shows a section along the line VIII-VIII through the sanitary napkin in Fig. 7.

### DETAILED DESCRIPTION OF PREFERRED EMBODIMENTS:

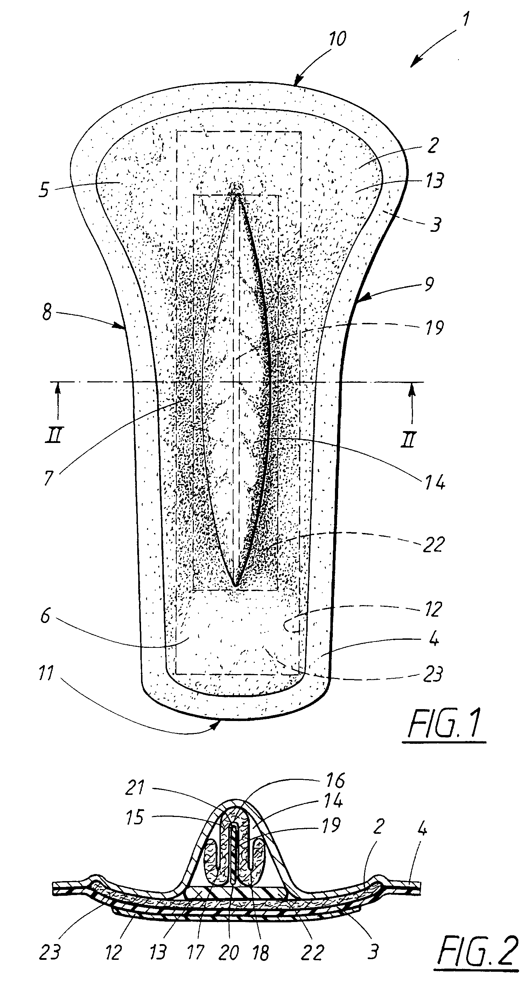

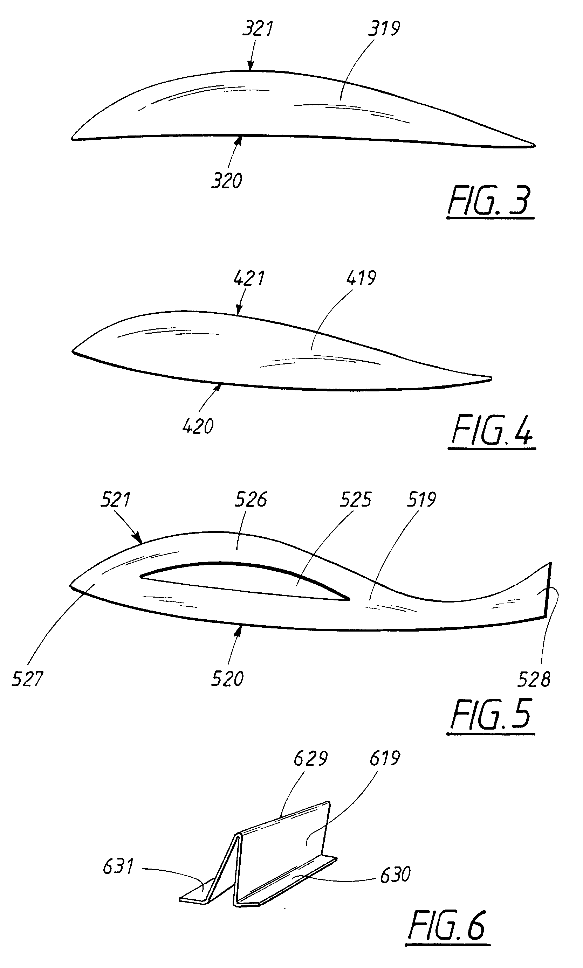

The sanitary napkin 1, shown in Figs. 1 and 2, comprises a liquid-pervious cover layer 2, arranged on the side of the sanitary napkin 1 which during use is intended to be facing the user. The liquid-pervious cover layer 2 suitably consists of soft, skin-friendly material. Examples of useful liquid-pervious cover materials are different types of non-woven fibre fabrics, so-called nonwoven materials. Other liquid-pervious cover materials used are perforated plastic films, scrims, knitted, crocheted or woven fabrics, and also combinations and laminates of the listed material types.

The sanitary napkin 1 further comprises a liquid-impervious cover layer 3, arranged at the side of the sanitary napkin 1 which during use is intended to be facing away from the user. As a rule, a thin, flexible plastic film is used as a liquid-impervious cover layer 3. However, it is also possible to use material layers which are originally liquid-pervious, but which are coated with a liquid-impervious material. In addition, other treatmentscan be used, such as thermo-calendering in order to fuse an originally liquid-pervious material into a substantially liquid-impervious layer. It is further possible to use nonwoven materials, or other textiles which are sufficiently dense, and the fibres of which are sufficiently hydrophobic, that they may function as a liquid barrier layer.

The two cover layers 2, 3 are mutually interconnected and form a protruding connection border 4 around the periphery of the sanitary napkin. The connection between the cover layers 2, 3 may be achieved by means of any previously known technique suitable for the purpose, such as gluing, welding or sewing.

The sanitary napkin 1 has almost a trapezium-shape, with a wider front portion 5 and a narrower rear portion 6, and an intermediate crotch portion 7. Furthermore, the sanitary napkin exhibits two longitudinal side edges 8, 9, and two transverse end edges 10, 11.

An attachment member 12, in the form of a longitudinal rectangular region of self-adhesive glue, is arranged on the surface of the liquid-impervious cover layer 3 which is facing away from the user. The attachment member 12 extends across the main part of the surface of the liquid-impervious cover layer 3, between the two end edges 10, 11. It is, of course, possible to use other glue patterns, such as longitudinal stripes, transverse regions, dots, circles, or other designs. Neither is the invention limited to adhesive attachment members, but frictional attachment and different kinds of mechanical attachment devices such as snap fasteners, clips, girdles, pants, or the like may be utilized if this is found to be convenient.

The sanitary napkin further comprises an absorbent body 13, for the acquisition of body fluids. The absorbent body 13 is enclosed between the two cover layers 2, 3, and has substantially the same shape in the plane of the sanitary napkin as the sanitary napkin as a whole.

The absorbent body 13 is composed of a plurality of components and exhibits a centrally arranged hump 14, extending in the longitudinal direction of the sanitary napkin, projecting from the surface which during use of the sanitary napkin is intended to be facing the user. The hump 14 comprises a first absorbent layer 15, which is pleated in the longitudinal direction of the sanitary napkin and which lends the hump 14 its shape. Accordingly, the previously shown first absorbent layer 15 has a longitudinal central fold 16, which forms the highest portion of the hump 14, i. e. the portion of the hump which stands out most from the surface of the sanitary napkin. On each side of the central fold 16, the first absorbent layer 15 exhibits an edge fold 17, 18, which is substantially parallel to the central fold 16 and at which the absorbent layer 15 is re-folded in a direction towards the highest portion of the hump 14. By means of the pleating of the first absorbent layer 15, the hump 14 is imparted a nearly triangular cross-sectional shape which conforms very well to the body shape of the user in the genital region.

In order to further adapt the hump 14 to the anatomy of the user and in order to facilitate bending of the sanitary napkin, the pleated absorbent layer 15 is, furthermore, bevelled in a direction towards the front and rear portions 5, 6 of the sanitary napkin, respectively. Thereby, the bevelling is somewhat steeper in a direction towards the rear portion 6, than in a direction towards the front portion 5.

The first absorbent layer 15 may consist of any material suitable for the purpose. For instance, the absorbent layer 15 may be a layer of fibre wadding. Such a layer may in itself be generally non-absorbent, but may contain particles or fibres of super-absorbent material. The term super-absorbent materials refers to polymers which are present in the form of fibres, flakes, particles, granular material, or the like and which are able to absorb several times their own weight of body fluid during swelling and formation of a gel.

Another type of material which may be used in the first absorbent layer 15 is the kind of absorbent material which is disclosed in WO 94/10956. This material is a dry-formed fibre layer with a high density and stiffness, which is used directly, without any preceding defibration. Another similar material, with properties especially adapted for blood absorption, is disclosed in WO 94/10953. The materials disclosed in WO 94/10956 and WO 94/10953 both have a comparatively high stiffness and thereby contribute to lending the hump a good shape stability. Furthermore, these fibre materials have very good absorption capacity. Upon absorption, the material swells somewhat in the thickness direction and thereby moulds itself to the available space in the crotch of the user. One advantage with this is that the hump 14 during use can adopt a uniquely adapted shape for each user. In this way both the leakage security and the comfort of the user are enhanced.

In order to avoid that the hump 14 during use is compressed in the thickness direction and loses its shape, a shape-stabilizing stiffening element 19 is arranged inside the central fold 16 in the first absorbent layer 15. The stiffening element 19 is advantageously made from some kind of stiff material which does not lose its stiffness when it is wetted. Particularly suitable materials for use as stiffening elements 19 are different types of plastics. The stiffening element 19 is constituted by a thin material piece, arranged approximately perpendicularly to the liquid-impervious cover layer 3. A first edge 20 on the stiffening element 19 has the same curvature as the longitudinal curvature on the surface of the sanitary napkin which is facing away from the user, while a second, opposing edge 21 follows the shape of the hump 14 along the central fold 16 in the first absorbent layer 15. In general, this implies that the first edge 20 is straight, or slightly convexly curved, while the second edge 21 is convexly curved and exhibits at least some portion with a larger curvature than the portion of the first edge which has the strongest curvature. In this context, curved edges should also be understood to include such edges which comprise two or several straight segments with mutually different inclinations.

Since a sanitary napkin is subjected to fairly large pressure forces during use, for example when a user sits down or rides a bicycle, the stiffening element 19 must be sufficiently stiff in order to essentially retain its shape during the entire period of use. Thereby, it is an advantage that the first absorbent layer 15 is arranged between the edge 20 of the stiffening element 19, closest to the liquid-pervious cover layer 2, and the stiffening element 19. This prevents the user from perceiving discomfort such as chafing or pressure from the stiffening element 19.

Furthermore, the stiffening element 19 serves as a folding edge for the first absorbent layer 15, whereby the central portion of the hump is shaped into a narrow, well-defined and shape-stable ridge. Thereby, a very good contact between the sanitary napkin and the body of the user is achieved, since the hump 14 can fit into the furrow between the labia pudendi of the user without discomfort to the user.

Since the stiffening element 19 is constituted by a material plate which is erected perpendicularly to the plane of the sanitary napkin 1, the liquid acquisition ability of the sanitary napkin is only insignificantly affected by the stiffening element 19. This is, of course, a crucial advantage, since the hump 14 is comparatively narrow because of the anatomically adapted design, and has a limited surface.

In order to avoid that the hump 14 is pressed back in a direction towards the liquid-impervious cover layer 3 during use, a reinforcement layer 22 is arranged on the inner side of the first absorbent layer 15. The reinforcement layer 22 is advantageously constituted by absorbent material which has been heavily compressed so that it is comparatively rigid. Suitable materials of this type are, for example, those which are disclosed in WO 94/10956 and WO 94/10953. Other useful materials are materials which have low extensibility and high tensile strength and which thus are able to resist deformation by pressure forces applied perpendicularly to the plane of the material. It is further possible to use materials with a low absorption capacity, or completely non-absorbent reinforcement layers 22, such as cardboard, rigid plastic inserts, or the like.

Closest to the liquid-impervious cover layer 3, a comparatively thin second absorbent layer 23 is arranged. The second absorbent layer 23 has a fairly limited absorption capacity and functions primarily as a reserve which is brought into use for absorption when the absorbent material in the hump 14 has been saturated with liquid. In addition, the second absorbent layer 23 has the ability to absorb small quantities of liquid which for some reason have ended up beside the hump 14, as well as liquid which happens to run out to the end portions 5, 6 of the sanitary napkin 1. Furthermore, the second absorbent layer 23 is intended to absorb perspiration from the user, so that the surface of the sanitary napkin and the skin of the user remain dry during use.

The absorbent material in the second absorbent layer 23 is suitably constituted by one, or several layers of a conventional absorbent material. Examples of useful materials are tissue layers, wadding layers of synthetic or natural fibres, layers of cellulose fluff pulp, absorbent foam layers, or the like.

In Figs. 3-5, stiffening elements 319, 419, 519 designed in different ways are shown. However, all the stiffening elements 319, 419, 519 are constituted by planar material pieces, for example, of plastic and generally have a thickness of between 0.2 and 2 mm.

The stiffening element 319 which is shown in Fig. 3 has a first, straight edge 320 intended in an absorbent article to be facing away from a user of the article. A second, curved edge 321 follows the shape of a shape-stable hump on the article and is intended to be facing towards the user of the article.

In Fig. 4 a stiffening element 419 is shown, wherein both the first and the second edge 420, 421 are curved. With such a stiffening element 419, the absorbent article conforms better to the outer curvature which is present in the region between the abdomen and the seat of the user.

In Fig. 5, a stiffening element 519 is shown having a cut-out portion 525 which substantially follows the shape of the stiffening element 519. It has been found that certain users may perceive a hump on an absorbent article as being too high, or too hard. However, by arranging one or several cut-out portions 525 in the stiffening element 519 it is possible to obtain a stiffening element 519 which is slightly compressible when pressure forces are applied on one or both edges 520, 521. Preferably, the material in the stiffening element is resilient, so that the stiffening element substantially recovers its original shape when the compression stops.

The cut-out portion 525 in the stiffening element 519 is largest where the stiffening element 519 is expected to be subjected to the greatest force action, namely at the central portion 526 of the stiffening element 519. Thereby, the stiffening element 519 may be compressed slightly more at its central, higher portion 526 than at the end portions 527, 528.

The stiffening element 519, shown in Fig. 5, furthermore has a raised rear end portion 528 which is shaped so as to provide an absorbent article with a rear hump intended to be able to be fitted into the furrow between the buttocks of the user.

In Fig. 6, a stiffening element 619 is shown having a design which is different from that of the stiffening elements in Figs. 3-5. The stiffening element 619 in Fig. 6 is constituted by a rectangular material sheet which has been folded or bent so that it exhibits a central longitudinal fold 629, which is intended to coincide with a central portion on a hump on an absorbent article. Furthermore, an edge portion of the material sheet, on both sides of the longitudinal fold 629, is folded out in order to form supporting edges 630, 631 which are intended to be facing away from a user of an absorbent article. The supporting edges 630, 631 function as force-absorbing and distributing members. Thereby, it is not completely necessary to use a special reinforcement layer of the kind which has been described in connection with Figs. 1 and 2 in order to avoid that a hump containing the stiffening element 619 is pressed in a direction away from the user.

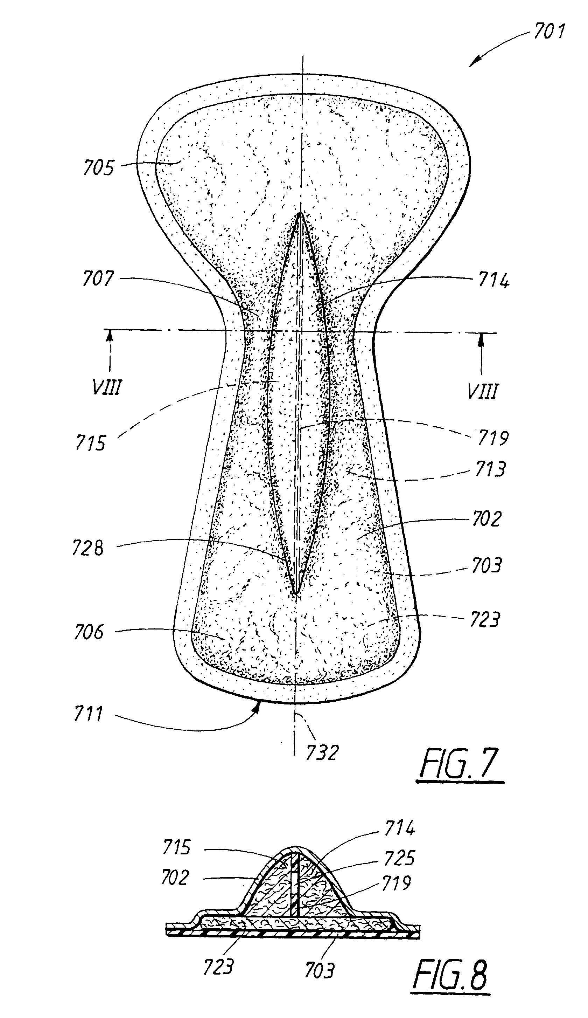

In Figs. 7 and 8, a further sanitary napkin 701 is shown, having a longitudinal hump 714 comprising a stiffening element 719 in accordance with the invention.

The sanitary napkin comprises a liquid-pervious cover layer 702 and a liquid-impervious cover layer 703, which together enclose an absorbent body 713. As in the sanitary napkin 1 shown in Figs. 1 and 2, the liquid-pervious cover layer 702 is constituted by a soft, flexible, skin-friendly material. The liquid-impervious cover layer 703, however, is constituted by a rigid material, preferably a relatively thick plastic layer.

The absorbent body 713 comprises a first absorbent part 715, which constitutes the longitudinal hump 714 of the sanitary napkin. The first absorbent part 715 consists of material with high absorption capacity, for example absorbent fibres such as cellulose fluff pulp, rayon or the like, with or without super-absorbent material, absorbent foam or some of the previously described absorbent fibre materials. All conceivable types of mixtures and combinations of materials may furthermore be used.

Centrally inside the longitudinal hump 714, a stiffening element 719, of the kind which is shown in Fig. 5, is arranged. The stiffening element is arranged along the longitudinal centre line 732 of the hump 714, generally perpendicularly to the liquid-impervious cover layer 703.

The sanitary napkin 701 is designed with a front portion 705 which is wider that the rear portion 706, and with a crotch portion 707 which is narrower that both the front portion and the rear portion. Since the liquid-impervious cover layer 703 is so rigid, it is essential that the sanitary napkin has a shape which is adapted to the anatomy of the user. It is of a particularly great importance that the width of the sanitary napkin, at least in the crotch portion 707, does not exceed approx. 4 cm.

The sanitary napkin further comprises an absorbent layer 723, arranged between the hump 714 and the liquid-impervious cover layer 703. The absorbent layer 723 can consist of some of the earlier listed absorbent materials and can, depending on the intended use of the sanitary napkin, be designed with greater or smaller absorption capacity.

The hump 714 of the sanitary napkin extends across the greater part of the rear portion 706, almost all the way to the rear edge 711 of the sanitary napkin. At the rear portion 706, the height of the stiffening element 719 increases gradually, as is evident from Fig. 5, whereby the sanitary napkin exhibits a rear hump 728. Such a rear hump 728 serves to seal against leakage backwards and, furthermore, constitutes a guiding member for keeping the sanitary napkin in a correct position in relation to the body of the user.

The invention should not be regarded as being limited to the herein described embodiments, but a number of further variants and modifications are conceivable within the scope of the claims. Furthermore, all conceivable combinations of the described embodiments are intended to be embraced by the invention.

## Claims

1. An absorbent article, such as a sanitary napkin, a panty-liner or an incontinence guard intended for female users, having a shape and size which permit it to be substantially accommodated in the crotch region of a pair of panties and having a longitudinal direction, a transverse direction and a thickness direction, wherein the article exhibits a liquid-pervious surface (2), and a liquid-impervious surface (3), and further an absorbent body (15, 23), arranged between the two surfaces (2, 3), and wherein a hump (14) is arranged at the liquid-pervious surface (2) of the article,
the hump (14) having an anatomically adapted shape and comprising a stiffening element (19) which extends between the liquid-pervious surface (2) and the liquid-impervious surface (3), **characterized in that** the stiffening element is essentially unyielding to compression in the thickness direction of the article both in a wet and in a dry state.

2. Absorbent article according to claim 1, **characterized in that** the stiffening element (19) is constituted by a rigid plastic layer having a thickness between 0.2 mm and 2 mm.

3. Absorbent article according to claim 1 or 2, **characterized in that** the stiffening element (619) is constituted by a rigid piece of material which is folded or bent and exhibits a fold (629) which is facing the liquid-pervious surface (2) and extends in the longitudinal direction of the article.

4. Absorbent article according to claim 3, **characterized in that** the stiffening element (619) exhibits at least one supporting edge (630, 631) formed by folding out an edge portion on the material piece and arranged at the liquid-impervious surface (3) of the article.

5. Absorbent article according to claim 1 or 2, **characterized in that** the stiffening element (19) is constituted by a planar piece of material, having a first edge (21) facing the liquid-pervious surface and a second edge (20) facing the liquid-impervious surface (3).

6. Absorbent article according to claim 5, **characterized in that** the stiffening element is arranged substantially perpendicularly between the two surfaces (2, 3) of the article.

## Patentansprüche

1. Absorptionsartikel, wie z.B. Hygienebinde, Slipeinlage oder Inkontinenzschutz, der für weibliche Benutzer vorgesehen ist, mit einer Form und Größe, die es ermöglichen, dass er im Wesentlichen in dem Schrittbereich einer Unterhose untergebracht wird, und der eine Längsrichtung, eine Querrichtung und eine Dickenrichtung aufweist, wobei der Artikel eine flüssigkeitsdurchlässige Oberfläche (2) und eine flüssigkeitsundurchlässige Oberfläche (3) und ferner einen Absorptionskörper (15, 23) aufweist, der zwischen den beiden Oberflächen (2, 3) angeordnet ist, und wobei ein Hügel (14) an der flüssigkeitsdurchlässigen Oberfläche (2) des Artikels angeordnet ist, wobei der Hügel (14) eine anatomisch angepasste Form aufweist und ein Versteifungselement (19) aufweist, das sich zwischen der flüssigkeitsdurchlässigen Oberfläche (2) und der flüssigkeitsundurchlässigen Oberfläche (3) erstreckt,
**dadurch gekennzeichnet, dass**
das Versteifungselement sowohl in einem nassen als auch in einem trockenen Zustand im Wesentlichen unnachgiebig gegen ein Zusammendrücken in der Dickenrichtung des Artikels ist.

2. Absorptionsartikel nach Anspruch 1,
**dadurch gekennzeichnet, dass**
das Versteifungselement (19) durch eine feste Plastikschicht mit einer Dicke zwischen 0,2 mm und 2 mm gebildet wird.

3. Absorptionsartikel nach Anspruch 1 oder 2,
**dadurch gekennzeichnet, dass**
das Versteifungselement (619) durch ein festes Materialstück gebildet wird, das gefaltet oder gebogen ist und einen Falz (629) aufweist, der zu der flüssigkeitsdurchlässigen Oberfläche (2) gerichtet ist und sich in der Längsrichtung des Artikels erstreckt.

4. Absorptionsartikel nach Anspruch 3,
**dadurch gekennzeichnet, dass**
das Versteifungselement (619) wenigstens einen Stützrand (630, 631) aufweist, der durch Ausfalten eines Randbereichs an dem Materialstück gebildet ist und an der flüssigkeitsundurchlässigen Oberfläche (3) des Artikels angeordnet ist.

5. Absorptionsartikel nach Anspruch 1 oder 2,
**dadurch gekennzeichnet, dass**
das Versteifungselement (19) durch ein ebenes Materialstück gebildet wird, das einen ersten Rand (21), der zu der flüssigkeitsdurchlässigen Oberfläche gerichtet ist, und einen zweiten Rand (20) aufweist, der zu der flüssigkeitsundurchlässigen Oberfläche (3) gerichtet ist.

6. Absorptionsartikel nach Anspruch 5,
**dadurch gekennzeichnet, dass**
das Versteifungselement im Wesentlichen senkrecht zwischen den beiden Oberflächen (2, 3) des Artikels angeordnet ist.

## Revendications

1. Article absorbant, tel qu'une serviette hygiénique, un protège-slip ou une protection contre l'incontinence pour utilisateurs de sexe féminin, ayant une forme et des dimensions qui lui permettent sensiblement d'être logé dans la région d'entrejambes d'un slip et ayant une direction longitudinale, une direction transversale et une direction d'épaisseur, l'article présentant une surface perméable (2) aux liquides et une surface imperméable (3) aux liquides, ainsi qu'un corps absorbant (15, 23), placé entre les deux surfaces (2, 3), et une bosse (14) étant disposée au niveau de la surface perméable (2) aux liquides de l'article, la bosse (14) ayant une forme anatomiquement adaptée et comprenant un élément raidisseur (19) qui s'étend entre la surface perméable (2) aux liquides et la surface imperméable (3) aux liquides, **caractérisé en ce que** l'élément raidisseur est essentiellement résistant à la compression dans la direction de l'épaisseur de l'article, aussi bien à l'état humide qu'à l'état sec.

2. Article absorbant selon la revendication 1, **caractérisé en ce que** l'élément raidisseur (19) est constitué d'une couche de plastique rigide ayant une épaisseur comprise entre 0,2 mm et 2 mm.

3. Article absorbant selon la revendication 1 ou 2, **caractérisé en ce que** l'élément raidisseur (619) est constitué d'un morceau de matière rigide qui est plié ou courbé et présente un pli (629) qui fait face à la surface perméable (2) aux liquides et s'étend dans la direction longitudinale de l'article.

4. Article absorbant selon la revendication 3, **caractérisé en ce que** l'élément raidisseur (619) présente au moins un bord de support (630, 631) formé en repliant une partie de bord sur le morceau de matière et disposé au niveau de la surface imperméable (3) aux liquides de l'article.

5. Article absorbant selon la revendication 1 ou 2, **caractérisé en ce que** l'élément raidisseur (19) est constitué d'un morceau de matière plat, ayant un premier bord (21) tourné vers la surface perméable aux liquides et un second bord (20) tourné vers la surface imperméable (3) aux liquides.

6. Article absorbant selon la revendication 5, **caractérisé en ce que** l'élément raidisseur est placé sensiblement perpendiculairement entre les deux surfaces (2, 3) de l'article.
